# EUROPEAN PATENT SPECIFICATION

(11) **EP 3 421 033 B1**
(45) Date of publication and mention of the grant of the patent: **27.07.2022**
(21) Application number: 18179796.0
(22) Date of filing: 06.10.2014
(51) Int. Cl.: A61K 9/24, A61K 31/4418, A61K 31/5377, A61P 31/12, A61P 31/18, A61P 43/00

(54) **HIV TREATMENT FORMULATION OF ATAZANAVIR AND COBICISTAT**
FORMULIERUNG ZUR HIV-BEHANDLUNG AUS ATAZANAVIR UND COBICISTAT
FORMULATION D'ATAZANAVIR ET DE COBICISTAT POUR LE TRAITEMENT DU VIH

(30) Priority: 07.10.2013 US 201361887574 P
(43) Date of publication of application: 02.01.2019
(62) Divisional of application: 14786421.9
(73) Proprietor: Bristol-Myers Squibb Holdings Ireland Unlimited Company, 6312 Steinhausen (CH)
(72) Inventor: KOO, Otilia May Yue, New Brunswick, New Jersey 08903 (US); NIKFAR, Faranak, New Brunswick, New Jersey 08903 (US); TAO, Jing, New Brunswick, New Jersey 08903 (US); KOTTALA, Niranjan Kumar, AP 530056 Visakhapatnam (IN); VARIA, Sailesh A., New Brunswick, New Jersey 08903 (US)
(74) Representative: Dehns

(56) References cited:
- WO-A2-2009/084036
- WO-A2-2011/127244
- US-A1- 2010 178 339
- RICHARD ELION ET AL: "Phase 2 study of cobicistat versus ritonavir each with once-daily atazanavir and fixed-dose emtricitabine/tenofovir df in the initial treatment of HIV infection", AIDS, vol. 25, no. 15, 1 September 2011 (2011-09-01), pages 1881-1886, XP055051636, ISSN: 0269-9370, DOI: 10.1097/QAD.0b013e32834b4d48
- Gilead Sciences International Ltd: "Tybost: International non-proprietary name: Cobicistat", European Medecines Agency , 25 July 2013 (2013-07-25), XP002732922, Retrieved from the Internet: URL:http://www.ema.europa.eu/docs/en_GB/do cument_library/EPAR_-_Public_assessment_re port/human/002572/WC500153016.pdf [retrieved on 2014-11-24]
- FDA: "Guidance for Industry Fixed Dose Combination and Co-Packaged Drug Products for Treatment of HIV", INTERNET CITATION, May 2004 (2004-05), XP002417855, Retrieved from the Internet: URL:http://www.fda.gov/oc/initiatives/hiv/ hivguidance.html [retrieved on 2007-01-31]
- PUJARI S ET AL: "Safety and long-term effectiveness of generic fixed-dose formulations of nevirapine-based HAART amongst antiretroviral-naive HIV-infected patients in India", INTERNET CITATION, 16 December 2003 (2003-12-16), XP002417803, Retrieved from the Internet: URL:http://libdoc.who.int/publications/200 3/a86263.pdf [retrieved on 2007-01-31]

## Description

### FIELD OF THE INVENTION

The invention is directed to formulations useful against HIV containing a two-drug combination of antiretroviral compounds. In particular, the invention is directed to a bilayer combination formulation of atazanavir and cobicistat as defined in claim 1. The invention is directed to a fixed dose combination tablet of atazanavir and cobicistat having good physical properties and low degradant levels, as well as efficacious delivery of the two active drug components.

### BACKGROUND OF THE INVENTION

HIV-1 (human immunodeficiency virus -1) infection remains a major medical problem, with tens of millions of people still infected worldwide at the end of 2013. The number of cases of HIV and AIDS (acquired immunodeficiency syndrome) has risen rapidly. In 2005, approximately 5.0 million new infections were reported, and 3.1 million people died from AIDS. Currently available drugs for the treatment of HIV include nucleoside reverse transcriptase (RT) inhibitors or approved single pill combinations: zidovudine (or AZT or RETROVIR^{®}), didanosine (or VIDEX^{®}), stavudine (or ZERIT^{®}), lamivudine (or 3TC or EPIVIR^{®}), zalcitabine (or DDC or HIVID^{®}), abacavir succinate (or ZIAGEN^{®}), Tenofovir disoproxil fumarate salt (or VIREAD^{®}), emtricitabine (or FTC - EMTRIVA^{®}), COMBIVIR^{®} (contains -3TC plus AZT), TRIZIVIR^{®} (contains abacavir, lamivudine, and zidovudine), EPZICOM^{®} (contains abacavir and lamivudine), TRUVADA^{®} (contains VIREAD^{®} and EMTRIVA^{®}); non-nucleoside reverse transcriptase inhibitors: nevirapine (or VIRAMUNE^{®}), delavirdine (or RESCRIPTOR^{®}) and efavirenz (or SUSTIVA^{®}), ATRIPLA^{®} (TRUVADA^{®} + SUSTIVA^{®}), and etravirine, and peptidomimetic protease inhibitors or approved formulations: saquinavir, indinavir, ritonavir, nelfinavir, amprenavir, lopinavir, KALETRA^{®} (lopinavir and Ritonavir), darunavir, atazanavir (REYATAZ^{®}) and tipranavir (APTIVUS^{®}) and cobicistat, and integrase inhibitors such as raltegravir (ISENTRESS^{®}) and dolutegravir (not yet approved), and entry inhibitors such as enfuvirtide (T-20) (FUZEON^{®}) and maraviroc (SELZENTRY^{®}).

Each of these drugs can only transiently restrain viral replication if used alone. However, when used in combination, these drugs have a profound effect on viremia and disease progression. In fact, significant reductions in death rates among AIDS patients have been recently documented as a consequence of the widespread application of combination therapy. However, despite these impressive results, 30 to 50% of patients may ultimately fail combination drug therapies. Insufficient drug potency, non-compliance, restricted tissue penetration and drug-specific limitations within certain cell types (e.g. most nucleoside analogs cannot be phosphorylated in resting cells) may account for the incomplete suppression of sensitive viruses. Furthermore, the high replication rate and rapid turnover of HIV-1 combined with the frequent incorporation of mutations, leads to the appearance of drug-resistant variants and treatment failures when sub-optimal drug concentrations are present. Therefore, novel anti-HIV agents exhibiting distinct resistance patterns, and favorable pharmacokinetic as well as safety profiles are needed to provide more treatment options.

One of the aforementioned drugs, atazanavir, has now established itself as a first-line antiretroviral in the treatment of HIV. It has the chemical formula C₃₈H₅₂N₆O₇, and the IUPAC name methyl *N*-[(1*S*)-1-{[(2*S*,3*S*)-3-hydroxy-4-[(2*S*)-2-[(methoxycarbonyl)amino]-3,3-dimethyl-*N*'-{ [4-(pyridin-2-yl)phenyl]methyl} butanehydrazido]-1-phenylbutan-2-yl]carbamoyl}-2,2-dimethylpropyl]carbamate. Its structural formula is set forth below:

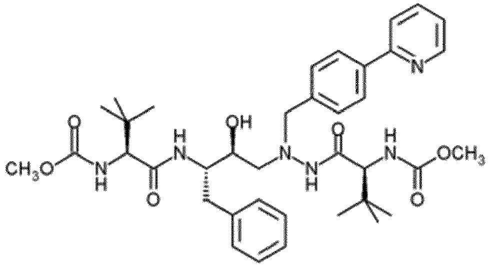

The compound is set forth and claimed in U.S. Patent No. 5,849,911. Atazanavir is distinguished from most other PIs in that it can be given once-daily (rather than requiring multiple doses per day) and has lesser effects on the patient's lipid profile (the amounts of cholesterol and other fatty substances in the blood). Like other protease inhibitors, it is used only in combination with other HIV medications.

Atazanavir is available from Bristol-Myers Squibb Company, Princeton, New Jersey, as REYATAZ^{®} (atazanavir sulfate). As used herein the terms "atazanavir" and "ATV" are synonymous and are intended to refer to atazanavir. As used herein the term "atazanavir sulfate" is intended to refer to atazanavir sulfate. The chemical name for atazanavir sulfate is (3S,8S,9S,12S)-3,12-Bis(1,1-dimethylethyl)-8-hydroxy-4,11-dioxo-9-(phenylmethyl)-6-[[4-(2-pyridinyl)phenyl]methyl]-2,5,6,10,13-pentaazatetradecanedioic acid dimethyl ester, sulfate (1:1). Its molecular formula is C₃₈H₅₂N₆O₇·H₂SO₄, which corresponds to a molecular weight of 802.9 (sulfuric acid salt).

REYATAZ^{®} is currently approved for use in combination with other antiretroviral agents for the treatment of HIV-1 infection. It is offered in capsule form in strengths of 100 mg (milligram), 150 mg, 200 mg, and 300 mg. In both the US and the European Union (EU), the approved ATV dose is 300 mg in combination with ritonavir ("RTV") 100 mg once daily with food for both treatment-naive and treatment-experienced patients with HIV infection. (see the REYATAZ^{®} (atazanavir sulfate) Capsules - Reyataz Package Insert http://packageinserts.bms.com/pi/pi_reyataz.pdf.

Atazanavir is rapidly absorbed (Tmax ∼2.5 hours) and demonstrates nonlinear pharmacokinetics (PK), with greater than dose-proportional increases in AUC (defined hereinafter) and Cmax (defined hereinafter) values over the dose range of 200 to 800 mg once daily (QD). Steady state is reached between 4 and 8 days, with an accumulation of approximately 2- to 3-fold. Atazanavir is given with food because it enhances bioavailability and reduces PK variability. The mean elimination half-life of ATV administered with a light meal in healthy subjects and HIV-infected adult subjects is approximately 7 hours at steady-state dose of 400 mg daily.

Also of significance is the drug known as cobicistat (also referred to herein as "COBI"). It is useful in the treatment of HIV, but does not specifically kill the virus. Rather, it has significant ability to inhibit liver enzymes that metabolize other medications used to treat HIV. Cobicistat is a potent inhibitor of cytochrome P450 3A enzymes, including the important CYP3A4 subtype. It also inhibits intestinal transport proteins, increasing the overall absorption of several HIV medications, including atazanavir. Cobicistat has the chemical formula C₄₀H₅₃N₇O₅S₂, and the structural formula: 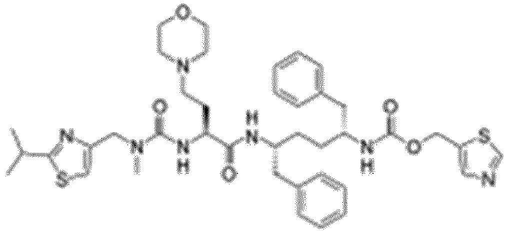 The compound is set forth and claimed in U.S. Patent No. 8,148,374.

Cobicistat has been approved for marketing by the US FDA as part of the fixed dose combination STRIBILD^{™} (elvitegravir, cobicistat, emtricitabine, and tenofovir disoproxil fumarate) to treat HIV infection.

The European Commission has also granted marketing authorization for once-daily TYBOST^{™} (cobicistat 150 mg tablets), a pharmacokinetic enhancer that boosts blood levels of certain HIV medicines. TYBOST^{™} is indicated as a boosting agent for the HIV protease inhibitors atazanavir 300 mg once daily and darunavir 800 mg once daily as part of antiretroviral combination therapy in adults with HIV-1 infection. Further information can be found at http://www.ema.europa.eu/ema and in the assessment report of the European Medicines Agency dated 25 July 2013 (*"Tybost: INN: Cobicistat*").

One additional issue associated with administration of HIV medications, including both atazanavir and cobicistat, is patient compliance. Because all HIV drugs must be taken as part of a combination regimen, there must be new and better ways to ensure that the patient actually takes each medication as prescribed. If there are too many separate pills to swallow, at too many time intervals, then dosing becomes inconvenient, and adherence to treatment is less likely. Similarly, if the pills or other dosage units themselves are difficult to swallow, then patient compliance could also be severely compromised.

Thus, what is now needed in the art are new, easily administered combination formulations containing potent antiretroviral drugs which are useful in the treatment against HIV. These new two drug formulations should be convenient and easy to administer, as well as be physically stable and have low degradant levels, and thereby provide efficacious dosing of important HIV medications.

In particular, stable, easily administered fixed dose combinations (FDCs) containing atazanavir and cobicistat are desired.

WO 2011/127244 and US 2010/178339 disclose tableted compositions comprising atazanavir sulfate optionally in combination with another active agent.

WO 2009/084036 discloses solid dosage forms of ritonavir and atazanavir/tipranavir.

Elion et al, AIDS, 2011, vol 25, no. 15, pages 1881-1886 discloses the results of a Phase 2 study of cobicistat versus ritonavir each with once-daily atazanavir and fixed dose emtricitabine/tenofivir in the initial treatment of HIV infection.

### SUMMARY OF THE INVENTION

By the present invention it is now possible to to reduce pill burden which has been associated with improved patient adherence in HIV therapy. In accordance with the present invention, a new FDC tablet, which may be comprised of 300 mg ATV and 150 mg COBI is provided. The FDC is a bilayer tablet which may be manufactured from ATV sulfate granulation for tablets which is preferably manufactured by a wet granulation process and COBI granulation preferably manufactured by a dry granulation process.

The bilayer tablets of the invention may be used in methods of treatment for patients infected with HIV.

The invention is directed to:
Item 1. A bilayer tablet composition comprising atazanavir, cobicistat and a pharmaceutically acceptable carrier, said composition comprising less than or equal to about 0.2% of degradant BMT-115982 or BMT-089290.
Item 2. A composition as in item 1, wherein said composition comprises less than or equal to about 4.0% total cobicistat impurities.
Item 3. A composition as in item 2, wherein said composition has less than about 2% total cobicistat impurities at about 12 months.
Item 4. A composition as in item 3, wherein said composition has less than about 1.4% cobicistat impurities at about 12 months.
Item 5. A composition as in item 4, wherein said composition does not crack upon release from a tablet press.
Also disclosed herein is a method of treating HIV infection in a patient, which comprises administering to said patient the composition as in item 5.

These and other objects of the invention will become apparent in the ensuing description and the appended claims.

### BRIEF DESCRIPTION OF THE DRAWINGS

Figure 1 is a cutaway side view of a comparative monolithic (single layer) tablet containing atazanavir sulfate and cobicistat.
Figure 2 is a graph showing cobicistat impurity after 8 weeks at 40°C and 75% relative humidity for a monolithic tablet (comparative), bilayer tablet of the invention, trilayer tablet (comparative), and cobicistat single agent tablet (comparative).
Figure 3 is a schematic illustration of the oxidative degradation of cobicistat.
Figure 4 is a cutaway side view of a comparative trilayer tablet containing atazanavir sulfate and cobicistat layers, with an inert excipient layer interposed between the two layers.
Figure 5 is a cutaway side view of the bilayer tablet containing atazanavir sulfate and cobicistat layers according to the invention.
Figure 6 is a graph of total cobicistat impurities for the atazanavir/cobicistat tablet under longer term conditions (12 months).

### DETAILED DESCRIPTION OF THE EMBODIMENTS

As part of the two drug formulation of the invention, the first active component is atazanavir. It may be utilized in its basic chemical form, but also as a salt or prodrug. In particular, atazanavir sulfate is preferred for use in the combination formulation herein.

The second active component is cobicistat, and may be utilized in any of its forms available to the skilled artisan.

The formulations of the present invention, according to all the various embodiments herein described, may be administered orally in dosage unit formulations containing non-toxic pharmaceutically acceptable carriers, excipients and diluents available to the skilled artisan. One or more adjuvants may also be included.

The pharmaceutical formulations of the invention are in the form of orally administrable tablets. Pharmaceutically acceptable carriers, excipients or diluents may be utilized in forming the tablets, and are those utilized in the art of pharmaceutical preparations.

As tablets, these formulations may contain, by way of non-limiting examples, microcrystalline cellulose, hydroxymethyl cellulose, hydroxyethyl cellulose, hydroxypropyl cellulose (HPC), hydroxypropyl methyl cellulose (HPMC), crospovidone, croscarmellose sodium, sodium starch glycolate and/or other available excipient polymers, as well as dicalcium phosphate, starch, stearic acid, magnesium stearate and lactose and/or other excipients, binders, extenders, disintegrants, diluents, and lubricants available to the artisan. In certain embodiments, micronized crystalline HCl salt may also be suitable.

In particular, the excipients microcrystalline cellulose, sodium starch glycolate, crospovidone, hydroxypropyl cellulose, and croscarmellose sodium, as well as stearic acid and magnesium stearate, are preferred for use herein.

To make the formulation of the invention, it is preferred to first separately prepare granulations of atazanavir and cobicistat, prior to forming the final atazanavir-cobicistat tablets. To make the atazanavir granulation, the active is first mixed with one or more of the aforementioned excipients in a suitable blender to blend the materials. Preferably, atazanavir (as atazanavir sulfate), is admixed with a first amount of microcrystalline cellulose, sodium starch glycolate, and crospovidone, as well as hydroxypropyl cellulose, and stearic acid. This mixture is wet granulated with water, then wet-milled, and the granules are then dried. Thereafter, a second amount of microcrystalline cellulose, sodium starch glycolate and crospovidone are added to the granules and further blended. Magnesium stearate is then added to the blend, and the final atazanavir granulation is collected in a suitable container.

Preferably, in one embodiment, the amount of atazanavir utilized will be such so as to deliver about 300 mg. of the drug (as the free base) in the final tablet formulation.

To prepare the cobicistat granulation as part of the atazanavir-cobicistat tablets, the cobicistat is preferably utilized with a suitable carrier, such as silicon dioxide. The cobicistat (on silicon dioxide) is then blended with a first amount of microcrystalline cellulose, as well as croscarmellose sodium. A first amount of magnesium stearate is then added, and the materials are blended. This mixture is then rolled, compacted, and screened to produce granules. A second amount of microcrystalline cellulose is then blended in, and finally a second amount of magnesium stearate. The final cobicistat granulation is then collected in a suitable container.

Preferably, in one embodiment, the amount of cobicistat utilized will be such so as to deliver about 150 mg. of the drug in the final tablet formulation.

To prepare the final tablets containing both atazanavir and cobicistat, both the atazanavir granulation mixture and the cobicistat granulation mixture are compressed into bilayer tablets using equipment available in the art. Preferably, a bilayer tooling process is utilized to make the tablets herein. Alternatively, a trilayer tooling process may be utilized (with two layers set up) to make the bilayer tablets herein.

It is further preferred that the tablets of the invention do not show visible (with the unaided eye) cracking at room temperature conditions (∼25° C and 1 atm.) within 24 hours of being released from the tablet press.

The tablets of the invention may have a hardness value within the range of about 28 - 39 SCUs.

It is further preferred that the tablets of the invention will have total cobicistat impurities (as hereinafter defined) of less than about 3.5%, when measured at about 8 weeks and 40°C and 75% relative humidity (RH). Even more preferably, the tablets will have total cobicistat impurities of less than about 3% under these conditions. Even more preferably, the tablets will have total cobicistat impurities of less than about 2.5% under these conditions.

In another embodiment, it is also preferred that the tablets of the invention will have total cobicistat impurities of less than about 2%, when measured at about 12 months and 5°C, and also when measured at about 12 months and 25° C and either 60% or 75% RH, and also when measured at about 12 months and 30° C and either 60% or 75% RH Even more preferably, the tablets will have total cobicistat impurities of less than about 1.4% under the foregoing conditions. Even more preferably, the tablets will have total cobicistat impurities of less than about 1.2% under these conditions.

In another embodiment, it is further preferred that the tablets of the invention will have specifications as follows: total cobicistat impurities of less than or equal to about 4.0%, and individual degradant levels as follows: BMT-115982 of less than or equal to about 0.2%; and BMT-0089290 of less than or equal to about 0.2%.

Once the compressed tablets are collected, an optional coating suspension may be preferably prepared. The core tablets may then be film-coated using this coating suspension. The final tablets will preferably contain about 20-45% (w/w) of atazanavir, based on the total weight of the formulation, and about 15-40% (w/w) of cobicistat (with carrier), based on the total weight of the formulation. It is also preferred that the atazanavir and cobicistat together comprise about 35-85% (w/w), based on the total weight of the final formulation.

Each of the two-component formulations herein set forth as part of the invention, can be administered orally to humans in a dosage range of about 1 to 100 mg/kg body weight one or more times daily, usually over an extended period, such as days, weeks, months, or even years. One preferred dosage range is about 1 to 10 mg/kg body weight orally per dose. Another preferred dosage range is about 1 to 20 mg/kg body weight orally per dose. Preferably, the formulations herein are compounded into once daily, once weekly or even once monthly or longer dosage forms, containing the 2 drug combinations of atazanavir and cobicistat herein set forth. More preferably, the final tablet formulation herein set forth will deliver about 300 mg. of atazanavir, and about 150 mg. of cobicistat.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy, as well as other possible factors.

Also disclosed herein is a method of treatment, for treating viral infections such as HIV infection and AIDS. The treatment involves administering to a patient in need of such treatment one or more of the bilayer tablets of the invention, which contain an antiviral effective amount of the two antiretroviral compounds atazanavir and cobicistat, together with one or more pharmaceutically acceptable carriers, excipients or diluents. As used herein, the term "antiviral effective amount" means the total amount of each active component of the composition and method that is sufficient to show a meaningful patient benefit, i.e., inhibiting, ameliorating, or healing of acute conditions characterized by inhibition of the HIV infection. When applied to the two-drug combination, the term refers to the combined amounts of the active ingredients that result in the therapeutic effect. The terms "treat, treating, treatment" as used herein and in the claims means preventing, ameliorating or healing HIV and/or diseases and conditions associated with HIV infection.

The following examples illustrate various aspects and embodiments of the invention, but should not be construed as limiting the scope thereof:

### EXAMPLE 1 - Manufacturing Process

### Manufacture of the atazanavir (ATV) granulation

The following is a description of the process for the manufacture of the ATV granulation for the ATV/COBI film-coated tablets. Materials may be prescreened, if necessary.
1. Add microcrystalline cellulose (intragranular portion), atazanavir sulfate, stearic acid, sodium starch glycolate (intragranular portion), crospovidone (intragranular portion), and hydroxypropyl cellulose to a suitable blender and blend the materials.
2. Wet granulate the preblend from Step 1 with water.
3. Wet mill the granules from Step 2 using a suitable Comil.
4. Dry the granules from Step 3 in a suitable fluid bed dryer.
5. Size the dry granules from Step 4 using a suitable Comil.
6. Add microcrystalline cellulose (extragranular portion), sodium starch glycolate (extragranular portion), and crospovidone (extragranular portion) to the granules from Step 5 and blend in a suitable blender.
7. Add magnesium stearate (extragranular portion) to the blend from Step 6 and blend in a suitable blender. Collect the ATV granulation into a suitable container.

### Manufacture of the cobicistat (COBI) Granulation

The following is the description of the process for the manufacture the cobicistat (COBI) granulation for ATV/COBI film-coated tablets. Materials may be prescreened, if necessary.
1. Add cobicistat on silicon dioxide, microcrystalline cellulose (intragranular portion), and croscarmellose sodium to a suitable blender and blend the materials.
2. Add magnesium stearate (intragranular portion) to a suitable blender that contains the preblend 1 from Step 1. Blend the materials.
3. Roller compact the preblend 2 mixture from Step 2. Mill the compacted material with a suitable size screen to produce granules.
4. Add microcrystalline cellulose (extragranular portion) to the granules from Step 3 and blend in a suitable blender.
5. Add magnesium stearate (extragranular portion) to the blend from Step 4 and blend in a suitable blender. Collect the COBI granulation into a suitable container.

### Manufacture of ATV/COBI Film-Coated Tablets

The following is the description of the process for the manufacture the ATV/COBI film-coated tablets from the ATV granulation and the COBI granulation.
1. Compress the ATV granulation and COBI granulation mixture into tablets. Collect the core tablets into bulk containers.
2. Prepare coating suspension by mixing Opadry Pink coating material in water.
3. Film coat the core tablets from Step 1 using coating suspension prepared in Step 2.
4. Package the film-coated tables into appropriate containers.

### EXAMPLE 2 Batch Size and Formula

The representative batch size and formula for the ATV/COBI film coated tablet are provided in Tables 1 and 2, respectively.

**Table 1: Representative Batch Size for ATV/COBI Film-Coated Tablet**

| Strength | Batch Size (kg) | Theoretical Number of Tablets |
|---|---|---|
| 300 mg (as the free base) Atazanavir sulfate / 150 mg Cobicistat | 140 | 133,333 |
| Batch size is for the uncoated tablets | | |

**Table 2: Representative Batch Formula for ATV/COBI Film-Coated Tablet**

| Component | Amount per Batch | | | | |
|---|---|---|---|---|---|
| | | Kg | | % w/w | |
| CORE TABLET | | | | | |
| ATV Granulation | | | | | |
| Intraflranular | | | | | |
| Atazanavir sulfate | | 45.56 | | | 56.95 |
| Stearic acid | | 2.24 | | | 2.80 |
| Microcrystalline cellulose | | 5.32 | | | 6.65 |
| Sodium starch glycolate | | 1.12 | | | 1.40 |
| Crospovidone | | 1.12 | | | 1.40 |
| Hydroxypropyl cellulose | | 1.12 | | | 1.40 |
| Water for injection | | q.s. | | | NA |
| Extragranular | | | | | |
| Microcrystalline cellulose | | 18.92 | | | 23.65 |
| Sodium starch glycolate | | 2.40 | | | 3.00 |
| Crospovidone | | 1.60 | | | 2.00 |
| Magnesium stearate | | 0.60 | | | 0.75 |
| ATV Granulation Batch Size | 80.00 | | 100.00 | | |

| Kg | % w/w | | | | |
|---|---|---|---|---|---|
| COBI Granulation | | | | | |
| Intragranular | | | | | |
| Cobicistat on silicon dioxide | 39.22 | 65.36 | | | |
| Microcrystalline cellulose | 10.88 | 18.14 | | | |
| Croscarmellose sodium | 3.00 | 5.00 | | | |
| Magnesium stearate | 0.30 | 0.50 | | | |
| Extragranular | | | | | |
| Microcrystalline cellulose | 6.00 | 10.00 | | | |
| Magnesium stearate | 0.600 | 1.00 | | | |
| COBI Granulation Batch Size | 60.00 | 100.00 | | | |
| Total Core Tablet Batch Size (Final blend) | 140.00 | | | | |
| FILM-COATING SUSPENSION Opadry | 4.20 | 3.00 | | | |
| Water for injection | q.s. | NA | | | |
| Total Tablet Batch Size (Film-coated tablets) | 144.20 | | | | |

| | | | | | |
|---|---|---|---|---|---|
| Note: The amount of atazanavir (as the free base) is theoretically equivalent to 87.8% of atazanavir sulfate salt. The amount shown is equivalent to 150 mg cobicistat (without silicon dioxide). The quantity of cobicistat on silicon dioxide is adjusted based on the drug content factor (DCF) for cobicistat on silicon dioxide with a concomitant adjustment in microcrystalline cellulose. q.s. = quantity sufficient; NA = Not applicable | | | | | |

### EXAMPLE 3 - Composition of ATV/COBI Film-Coated Tablet with 300 mg. (as the free base) atazanavir sulfate/150 mg. cobicistat is set forth below in Table 3:

**Table 3: Composition of ATV/COBI Film-Coated Tablet**

| Component | Quality Standard | Function | Quantity per Tablet (mg) | |
|---|---|---|---|---|
| | | | mg | % w/w |
| Atazanavir Sulfate Layer | | | | |
| Atazanavir Sulfate | In-house | Active | 341.70 | 56.95 |
| Stearic Acid | NF, Ph.Eur. | Lubricant | 16.80 | 2.80 |
| Microcrystalline Cellulose | NF, Ph.Eur. | Filler | 181.80 | 30.30 |
| Sodium Starch Glycolate | NF, Ph.Eur. | Disintegrant | 26.40 | 4.40 |
| Crospovidone | NF, Ph.Eur. | Disintegrant | 20.40 | 3.40 |
| Hydroxypropyl Cellulose | NF, Ph.Eur. | Binder | 8.40 | 1.40 |
| Magnesium Stearate | NF, Ph.Eur. | Lubricant | 4.50 | 0.75 |
| Water for Injection | USP | Granulating Liquid | NA | - |
| Weight of Atazanavir Layer | | | 600.00 | 100.00 |
| Cobicistat Layer | | | | |
| Cobicistat on silicon dioxide | In-house | Active | 294.12 | 65.36 |
| Microcrystalline Cellulose | NF, Ph.Eur. | Filler | 126.63 | 28.14 |
| Croscarmellose Sodium | NF, Ph.Eur. | Disintegrant | 22.50 | 5.00 |
| Magnesium Stearate | NF, Ph.Eur. | Lubricant | 6.75 | 1.50 |
| Weight of Cobicistat Layer | | | 450.00 | 100.00 |

| Table 3 (continued): Coating Composition of ATV/COBI Film-Coated Tablet | | | | |
|---|---|---|---|---|
| Component | Quality Standard | Function | Quantity per Tablet (mg) | |
| | | | mg | % w/w |
| Opadry | NC | Coating agent | 31.5 | - |
| Water for Injection | USP | Vehicle for coating suspension | NA | - |
| Total Tablet Weight (film-coated tablets) | | | 1081.50 | |

The amount of atazanavir (as the free base) is theoretically equivalent to 87.8% of atazanavir sulfate salt. The quantity is based on the theoretical assay "as is" of 87.8%; 341.7 mg of atazanavir sulfate is equivalent to 300 mg of atazanavir. Adjustments may be made based on the actual assay "as is" of the drug substance batch that will be used for drug product manufacture.

Cobicistat drug substance is cobicistat on silicon dioxide. The amount shown is equivalent to 150 mg cobicistat (without silicon dioxide). The quantity of cobicistat on silicon dioxide is adjusted based on the drug content factor (DCF) for cobicistat on silicon dioxide with a concomitant adjustment in microcrystalline cellulose.

(NC = Non-compendial NF = National Formulary Ph.Eur. = European Pharmacopoeia USP = United States Pharmacopeia)

### EXAMPLES 3A/3B - Proposed Specifications for ATV/COBI Fixed-Dose Combination Film-Coated Tablet (FCT) are set forth below in Table 3A:

The identity and quality of each batch of ATV/COBI FCT are assessed and confirmed according to the proposed specifications provided in Table 3A below. Designations "G" and "S" that follow the analytical method numbers on the specifications are abbreviations for the general and specific methods, respectively.

The specification includes all the quality attributes of the drug product (appearance, assay, content uniformity, impurities/degradants, and dissolution) that affect the manufacturing and quality of the drug product. The microbial limits are also part of the specification.

All methods were validated to demonstrate that they are suitable for their intended use to monitor the quality of ATV/COBI FCT. Analytical methods were validated for specificity, linearity, accuracy, precision, and robustness, according to current guidelines. The tests for uniformity of dosage unit reflect the harmonized pharmacopeial general chapters.

Summary of the batch analyses data of investigational and registrational stability (including clinical trials (BE study)) studies and commercial-scale batches for ATV/COBI FCT is provided in Table 3B. These batches are considered representative of the proposed commercial process.

The batch analysis data summarized in Table 3B along with the stability data were used to justify the proposed specifications. The justification of the proposed specifications and analytical method are provided below.

**Table 3A: Proposed Specifications for ATV/COBI FCT**

| Test | Method Number (Description) | Acceptance Criterion |
|---|---|---|
| Description | Not Applicable | An oval, biconvex, pink film coated tablet debossed with 3641 on one side and a plain face on the other side. |
| Identification HPLC | 95015164 (S) | The retention time of the two major peaks in the sample chromatogram correspond to those of the standard chromatogram. |
| IR - ATR | 95015471 (S) | |
| | | The spectra of sample conforms to the reference spectra. |
| Uniformity of Dosage Units | | Complies with harmonized compendial requirements (USP/EP/JP/ChP) |
| Atazanavir | 95015164 (S) HPLC 356X (G) | |
| Cobicistat | 95015164 (S) HPLC 356X (G) | |
| Assay (% of Label Claim) | | |
| Atazanavir | 95015164 (S) HPLC | 90.0% - 110.0% |
| Cobicistat | 95015164 (S) HPLC | 90.0% - 110.0% |
| Impurities/Degradants | 95015319 (S) HPLC | |
| GS-9612 | | ≤0.8% |
| GS-465430 | | ≤1.2% |
| GS-9398 | | ≤0.5% |
| GS-445739 | | ≤0.5% |
| GS-9454 | | ≤0.4% |
| GS-344433 | | ≤0.4% |
| BMT-111068 & BMT-111069 | | ≤1.5% |
| BMT-115982 | | ≤0.2% |
| BMT-089290 | | ≤0.2% |
| Individual Unspecified Degradant | | ≤0.2% |
| Total Cobicistat impurities/degradants | | ≤4.0% |
| Total Atazanavir impurities/degradants | | ≤1.0% |
| Dissolution Atazanavir | 0311 (G), 95015163 (S) | ≥75(Q) in 30 minutes |
| Cobicistat | 0311 (G), 95015163 (S) | ≥80(Q) in 15 minutes |
| Microbial Limits | | |
| Total Aerobic Microbial Count | 5450A(G), 95015304(S) | ≤ 1000 CFU/gram |
| Total Yeasts and Molds Count | 5450A(G), 95015304(S) | ≤ 100 CFU/gram |
| | | Absent in 1 gram |
| E.coli | 5450A(G), 95015304(S) | |

**Table 3B: Batch Analyses Summary for ATV/COBI FCT Batches**

| Test | Range of Results (n = 13)^{a} |
|---|---|
| Appearance (Description) | An oval, biconvex, pink film-coated tablet debossed with BMS on one side and 468 on the other side^{b}. |
| | An oval, biconvex, pink film-coated tablet debossed with 3641 on one side and a plain face on the other side^{c}. |
| Identification | The results for all batches were 'complies' |
| HPLC | |
| Assay (% of label claim) | |
| ATV | 97.3-101.7 |
| COBI | 98.7-103.6 |
| Uniformity of Dosage Unit | |
| ATV | |
| Content Uniformity | Complies |
| Acceptance Value | 1.8-8.6 |
| %RSD | 1.0-3.6 |
| COBI | |
| Content Uniformity | Complies |
| Acceptance Value | 2.0-10.8 |
| %RSD | 0.8-3.8 |
| Impurities/Degradants (%) | |
| GS-9612 | <0.05-0.10 |
| GS-465430 | <0.05-0.11 |
| GS-9398 | <0.05 |
| GS-445739 | <0.05 |
| GS-9454 | <0.05-0.05 |
| GS-344433 | <0.05 |
| BMT-111068 & BMT-111069 | <0.05-0.16 |
| BMT-115982 | <0.05 |
| BMT-089290 | <0.05 |
| Individual Unspecified^{d} (%) | <0.1 |
| Total ATV impurities/degradants^{e} (%) | <0.05 |
| Total COBI impurities/degradants^{f} (%) | 0.78-1.38 |
| Dissolution | |
| ATV (Average % dissolved in 30 min) | ≥84 |
| COBI (Average % dissolved in 15 min) | ≥96 |
| Microbial Limits | |
| Total Aerobic Microbial Count | NT - <100 |
| Total Yeasts and Molds Count | NT - <100 |
| E.coli | NT - Absent |

| | |
|---|---|
| ^{a}Three investigational stability, three registrational stability, and seven commercial-scale batches. Justification of specification was proposed based on 10 batches-three investigational stability, three registrational stability, and four commercial-scale batches. All batches meet the proposed acceptance criteria. ^{b}Appearance/description for the investigational and registrational stability batches. ^{c}Appearance/description for the commercial product. ^{d}Any individual unspecified impurity of unknown origin will be reported against the COBI peak. ^{e}Sum of ATV process impurities and ATV degradants; Drug substance process related impurities are not specified individually however, they are captured under "Total ATV impurities/degradants". ^{f}Sum of COBI process impurities and COBI degradants; Drug substance process related impurities are not specified individually however, they are captured under "Total ATV impurities/degradants". | |

### Characterization of Impurities

No ATV related impurities/degradants are observed in ATV/COBI FCT. GS-465430, GS-9612, GS-445739, GS-9398, GS-344433, GS-9454 are cobicistat drug substance related impurities, which are also drug product degradants. BMT-111068, BMT-111069, BMT-115982, and BMT-089290 are additional cobicistat related drug product degradants.

### Justification of Specification

The justifications for the proposed specifications (presented in Table 3A) are supported by the following:
- A total of 10 batches, which includes 3 investigational stability, 3 registrational stability (including the batch used in the bioequivalence study, 2J73499), and 4 commercial-scale batches (batch # MTXX, MTXY, MYVB, and MYVC), manufactured using the proposed commercial formulation and process and packaged in the proposed commercial packaging (HDPE bottle), were used to support the proposed specifications for ATV/COBI FCT. A summary of the batch analysis data is presented in Table 3B.
- Nine month stability data from 3 registrational stability batches, 15-month stability data from 2 investigational stability batches and 12-month stability data from one investigational stability batch at 30°C/75%RH were used to support the proposed specifications for ATV/COBI FCT. *Stability.* The available data show that the ATV/COBI FCT drug product packaged in HDPE bottle, is stable at the long-term (5°C, 25°C/60%RH and 30°C/75%RH) as well as accelerated (40°C/75%RH) storage conditions used in the study.

During the formulation process it was unexpectedly discovered that the bilayer formulation was highly suitable in terms of stability, manufacturability and projected ease of administration. The formulation route itself was not straightforward, nor was it simple. It was the result of significant concerted effort.

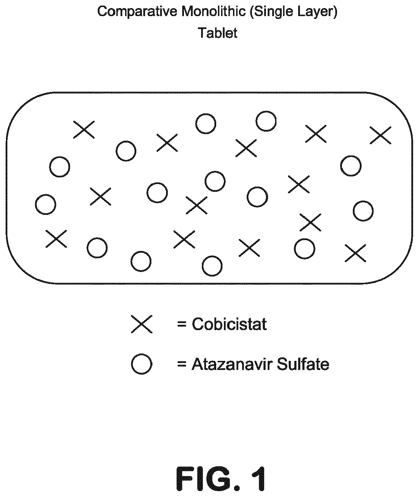

The first attempt involved formulation of a comparative monolithic (single layer) tablet wherein both the actives and the excipients were fairly evenly dispersed throughout the entire tablet (not including the tablet coating). This is conceptually illustrated in Figure 1. This project presented theoretical challenges. Atazanavir sulfate is acidic, while cobicistat is susceptible to acidic degradation pathways. However, it was unknown the extent of the influence of atazanavir sulfate on cobicistat in the matrix of monolithic formulation with excipients. Despite this, it was fully expected that the two APIs atazanavir and cobicistat, in conjunction with the microcrystalline cellulose and the other listed excipients, could be formulated into a stable monolithic tablet.

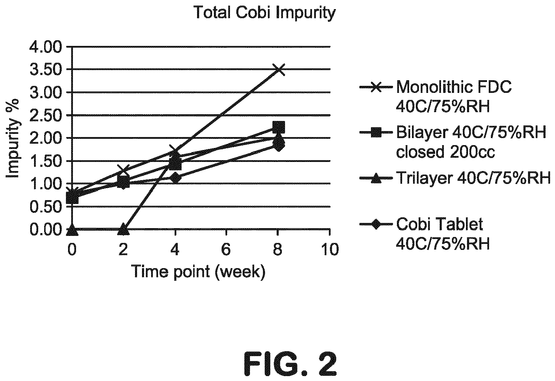

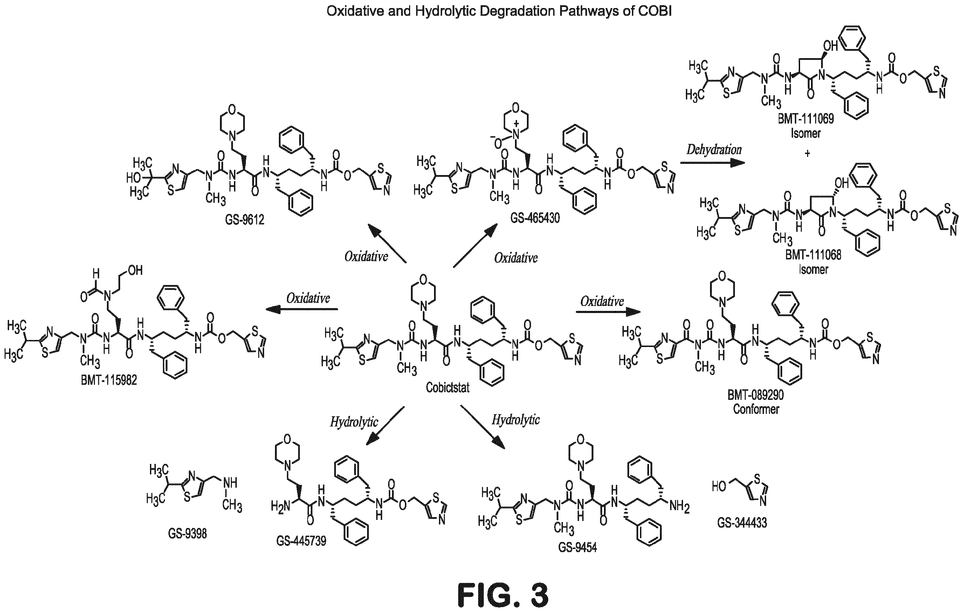

Several monolithic blends were tested that were largely equivalent, and which all yielded the desired dose ratio of atazanavir active to cobicistat of approximately 300 mg/ 150 mg. However, the final monolithic tablet formulations proved to be considerably worse in cobicistat stability than expected, despite the presence of the excipients. As the graph in Figure 2 illustrates, when total cobicistat impurity percentage was measured against time, it was discovered that total impurity % reached approximately 3.50% after 8 weeks in closed bottles at 40 °C/75% relative humidity. This was deemed to be unacceptable, since this level was more than 2-3 fold higher than the comparative cobicistat single agent tablet (also shown in Figure 2) used as a reference. The large upward slope to the impurity line in Figure 2 indicates that the trend itself is also unfavorable, when projected to what happens when the formulation is stored under normal room storage conditions. This result indicated that monolithic tablets would not be utilized as a final formulation. (Cobicistat impurity was measured as impurity/degradation peaks above a threshold level of 0.05% either identified as cobicistat impurities or degradation products through a reference standard, or any other peaks (unknown) not identified as atazanavir impurity peaks. For example, Figure 3 illustrates how the reference degradation products are formed from cobicistat.)

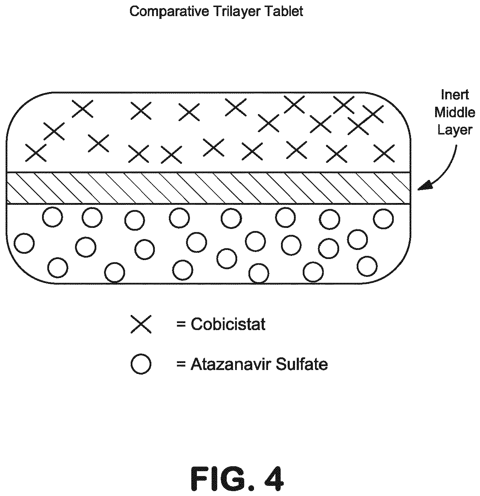

The problem thus remained as to how to form a dosage form with acceptable cobicistat stability. It was also proposed to separate the two layers of drugs by having an inert third layer imposed between the two respective layers of active compounds. This is conceptually illustrated in Figure 4. Thus, a comparative trilayer formulation was developed in which about 250 mg. of inert excipient material was spaced as a middle or second layer between the layers of atazanavir sulfate formulation (600 mg.) and cobicistat (on SiO₂) formulation (450 mg.). Various compositions of the middle layer such as lubricated microcrystalline cellulose (MCC), and MCC with SYLOID^{®} silica excipients in amounts up to 20% by weight respectively (silica weight was relative to the middle layer), were tried. Another middle layer formulation was made up of mixture of approximately equal portions of lubricated microcrystalline cellulose and lactose monohydrate. A small amount (about 0.5 - 1 weight %) of magnesium stearate was also included to lubricate the inert middle layer formulations. Overall, the inert layer would function to separate and thereby significantly minimize or eliminate interactions between the two main compounds.

Tablets were then formed using standard trilayer tooling and methodology. As can be seen in Figure 2, the trilayer tablets had a significantly enhanced cobicistat stability and reduced degradation profile in that there were only about 2% cobicistat impurities measured after 8 weeks in closed bottles at 40 °C/75% relative humidity. This was very comparable to the cobicistat single agent tablet, and was a greatly improved result compared to the monolithic atazanavir/cobicistat fixed-dose combination tablets, which had cobicistat impurity levels of approximately 3.50% after 8 weeks.

However, there was an unexpected problematic issue with the trilayer tablets. Despite utilizing accepted methodology and protocol, significant cracks were often observed between the atazanavir layer and the middle inert layer for many of the inert layer formulations. These typically developed upon ejection from the tablet press. The cracks were often immediately visible to the unaided eye. What was most problematic, however, was that the appearance of the cracks, as well as their size and the total number of cracks per tablet, was very unpredictable from one trilayer batch to the next.

Larger surface area tooling was then employed, and this method typically generated trilayer tablets without cracks under similar compression forces. This was possibly due to larger cup area and/or lower cup depth. But this presented an additional problem. Larger surface area tablets were wider, and so there was significant concern that these would pose a greater swallowing risk, even though the total trilayer tablet weight was the same. Many people who are taking HIV medications are not only immune compromised, but may also be physically frail and debilitated as well. Furthermore, this therapy is meant to be long-term (up to several years). It could be too much of an effort, and thereby too much of a risk, to have these patients attempt to swallow these trilayer tablets with wider surfaces. If the patients could not remain compliant, then the medication would not serve its purpose.

Thus, after attempting to form a suitable trilayer formulation, it was determined that the various approaches were not going to yield an acceptable dosage form.

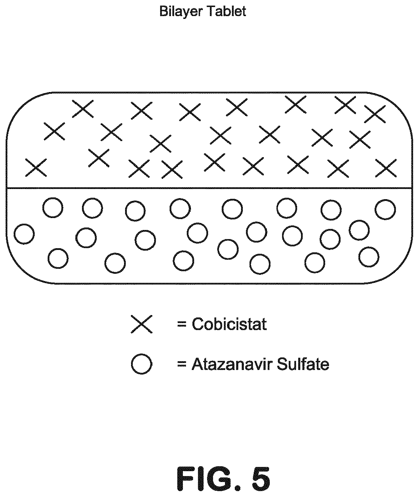

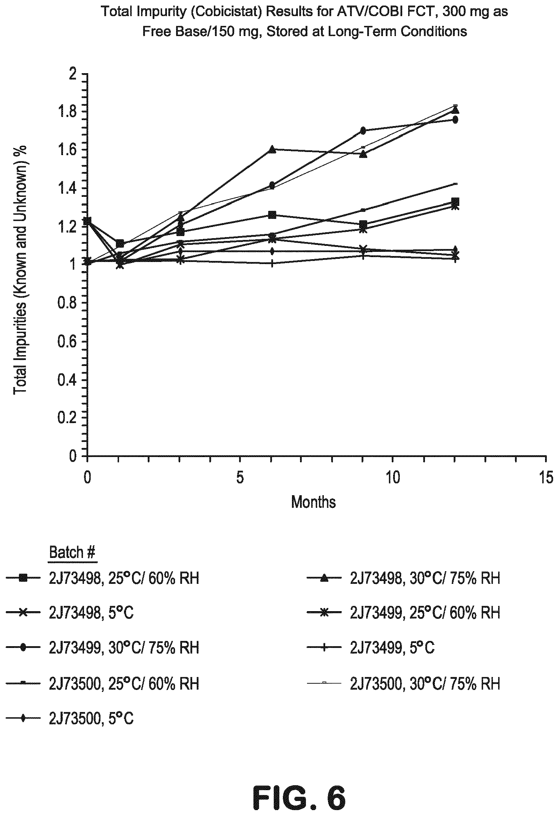

Ultimately, the bilayer tablets formed in accordance with the procedures hereinabove set forth as the invention, were surprisingly found to be highly efficacious and were pursued herein. The bilayer tablet (uncoated version) is conceptually illustrated in Figure 5. Even though there was physical contact between the two chemically incompatible compounds atazanavir sulfate and cobicistat at the layer interface within the tablet, the results indicated that the degradation profile was very similar to that of the trilayer tablets. As Figure 2 indicates, the bilayer formulation's degradation profile was very close to that of the trilayer formulation after 8 weeks. The bilayer tablet's profile was also much better than that of the monolithic atazanavir/cobicistat tablet and comparable to the cobicistat single agent tablet. This also indicated that within the established formulation matrix with the selected excipients, atazanavir and cobicistat were very compatible. Importantly as well, there was no cracking observed with the bilayer tablets, unlike with the trilayer tablets. Just as importantly, as Example 4 below further illustrates, this bilayer formulation also provided bioequivalent atazanavir and cobicistat exposures when compared to co-administration of the single agent atazanavir capsule and cobicistat tablet. Thus, the bilayer tablet was unexpectedly the formulation which proved to be the most viable and suitable for development herein. As Figure 6 also illustrates, the tablets of the invention were highly stable over the long term with total cobicistat impurities at 12 months of less than about 2% (and lower) when measured at varying temperature and relative humidity conditions shown with the graph.

### EXAMPLE 4 - Atazanavir/Cobicistat Fixed-Dose Combination Bioequivalency Study

A study was conducted to demonstrate the bioequivalence of ATV in a FDC of ATV/COBI (300/150 mg) as compared to a 300 mg ATV capsule co-administered with a 150 mg COBI tablet when administered with a light meal.

The study was conducted as an open-label, single-dose, 5-period, 5-treatment, randomized crossover study in healthy subjects. Subjects were equally randomized to one of eight treatment sequences (ABCDE, ABDCE, BACDE, BADCE, ABCD, ABDC, BACD or BADC). Subjects underwent screening evaluations to determine eligibility within 21 days prior to randomization. Subjects were admitted to the clinical facility 1 day prior to dosing on Day 1. Subjects received a 300 mg ATV capsule co-administered with a 150 mg COBI tablet or FDC tablet of ATV/COBI (300/150 mg) following a light meal (Treatment A or B, respectively) according to the assigned treatment sequences on Day 1 and Day 8. On Day 15 and Day 22, subjects received a 300 mg ATV capsule co-administered with a 150 mg COBI tablet or FDC tablet of ATV/COBI (300/150 mg) under fasted conditions (Treatment C or D, respectively) according to the assigned treatment sequences. On Day 29, some subjects, according to the assigned sequences, received a FDC tablet of ATV/COBI (300/150 mg) following a high fat meal (Treatment E). There was a 7-day washout period between each treatment.

Blood samples for pharmacokinetic (PK) analysis were collected at scheduled times throughout the study. Subjects were monitored for safety via physical examinations (PEs), electrocardiograms (ECGs), physical measurements, vital signs measurements, clinical laboratory testing and adverse event (AE) reporting, and some subjects, according to assigned sequences, were discharged from the study at the end of Period 4 (Day 24) and the remaining subjects continued to Period 5 and were discharged at the end of Period 5 (Day 31). Subjects remained confined in the clinical pharmacology unit (CPU) from Day -1 until discharged from the study.

The study design schematic is presented below.

**Study Design Schematic**

| | | | | | | | | | | |
|---|---|---|---|---|---|---|---|---|---|---|
| Days -21 to -1 | Period 1 and Period 2^{a} | | | Washout up to Day 14 | Periods 3 and 4^{b} | | | Washout up to Day 28 | Period 5^{c} | |
| | Day 1 | Washout up to Day 7 | Day 8 | | Day 15 | Washout up to Day 21 | Day 22 | | Day 29 | Day 31 |
| S& E^{d} | Treatment A or B | | Treatment B or A | | Treatment C or D | | Treatment D or C | | Treatment E | Discharge |

| | | | | | | | | | | |
|---|---|---|---|---|---|---|---|---|---|---|
| ^{a}Subjects randomized prior to Day 1 dosing ^{b}Subjects discharged at the end of Period 4 (Day 24) based on the assigned treatment sequence ^{c}Subjects discharged at the end of Period 5 (Day 31) based on the assigned treatment sequence ^{d} Screening & Enrollment Treatment A (comparative): Atazanavir 300 mg (Capsule) + 150 mg Cobicistat (Tablet) co-administered following a light meal Treatment B: Atazanavir 300 mg/150 mg Cobicistat - FDC (Tablet) administered following a light meal Treatment C (comparative): Atazanavir 300 mg (Capsule) + 150 mg Cobicistat (Tablet) co-administered under fasted conditions Treatment D: Atazanavir 300 mg/150 mg Cobicistat - FDC (Tablet) administered under fasted conditions Treatment E: Atazanavir 300 mg/150 mg Cobicistat - FDC (Tablet) administered following a high fat meal | | | | | | | | | | |

In this study, the atazanavir 300 capsule (Treatment A and C) was administered as Reyataz (atazanavir sulfate) obtained from Bristol-Myers Squibb Company, Princeton, NJ and the 150 mg cobicistat tablet (Treatment A and C) was administered as cobicistat on silicon dioxide obtained from Gilead Sciences, Foster City, CA. The FDC tablets (Treatments B, D and E) were prepared as described in Examples 1-3 above.

This Phase III trial compared the efficacy and safety of ATV 300 mg + COBI 150 mg relative to ATV 300 mg + RTV (ritonavir) 100 mg (85% vs. 87% virologic suppression, respectively). The bioequivalence of ATV and relative bioavailability of COBI in an FDC vs. ATV and COBI coadministered individually after a light meal was assessed.

This randomized, open-label, cross-over study in 64 healthy subjects assessed 48-hour ATV and COBI plasma concentration-time profiles after single doses of an FDC of ATV 300 mg/COBI 150 mg or ATV 300 mg and COBI 150 mg co-administered as individual agents. Treatments were administered after a light meal and followed by a 7-day washout between each period. Pharmacokinetic (PK) parameters assessed were Cₘₐₓ, maximum plasma concentration; AUC(INF), area under the concentration-time curve to infinity; and AUC(0-T), area under the curve from zero to the last time point with measurable concentration. Bioequivalence for ATV was established if the 90% confidence intervals (CIs) for the FDC vs. individual administration geometric mean ratios (GMRs) fell within the predefined limits of 0.80-1.25 for all PK parameters.

Representative light meals and heavy meals were as follows in Tables 4a and 4b.

**Table 4a: Representative Light Meal**

| Food Item | Calories (kcal) | Fat (g) | Carbohydrates (g) | Protein (g) |
|---|---|---|---|---|
| 2 slices of white bread | 129 | 1.8 | 24.0 | 4.0 |
| 1 teaspoonful low fat margarine | 24 | 2.8 | trace | trace |
| 1 tablespoon jam | 56 | trace | 13.8 | trace |
| 5 oz apple juice | 73 | 0.2 | 18.1 | 0.1 |
| 5 oz skim (non-fat) milk | 54 | 0.3 | 7.4 | 5.2 |
| Total Grams (g) | -- | 5.1 | 63.3 | 9.3 |
| Total Calories (kcal) | 336 | 46 | 253 | 37 |
| % of Total Calories | 100 | 14 | 75 | 11 |

| | | | | |
|---|---|---|---|---|
| Source: US Department of Agriculture Nutrient Database for Standard Reference, Release 25 (2012 | | | | |

**Table 4b: Representative High Fat and Calorie Meal**

| Food Item | Calories (kcal) | ^{a}Fat | Carbohydrate | ^{a}Protein (g) |
|---|---|---|---|---|
| 2 eggs fried | 180 | 14.1 | 0.8 | 12.5 |
| 2 slices of white bread toasted | 129 | 1.8 | 24.0 | 4.0 |
| 1 tablespoon butter | 102 | 11.5 | Trace | 0.1 |
| 1 tablespoon jam | 56 | Trace | 13.8 | Trace |
| 3 strips of bacon fried | 126 | 9.6 | 0.4 | 9.1 |
| 4 ounces of hash brown potatoes | 299 | 14.1 | 39.7 | 3.4 |
| 8 fluid ounces (237 mL) of whole milk | 146 | 7.9 | 11.0 | 7.9 |
| Total (g) | - | 59.0 | 89.7 | 37.0 |
| Total Calories (kcal) | 1038 | 531 | 359 | 148 |
| % of Total Calories | 100 | 51 | 35 | 14 |

| | | | | |
|---|---|---|---|---|
| ^{a}Source: US Department of Agriculture Nutrient Database for Standard Reference, Release 25 (2012) | | | | |

The treatment administration was as follows in Table 5.

**Table 5: Treatment Administration**

| Treatment | Total Daily Dose | Capsule/Tablet Strength | Number of Capsules/Tablets |
|---|---|---|---|
| A and C | 300 mg ATV capsule | 300 mg | 1 capsule |
| | 150 mg cobicistat tablet | 150 mg | 1 tablet |
| B, D and E | 300 mg ATV/150 mg cobicistat tablet | 300 mg/150 mg | 1 tablet |

The pharmacokinetics of ATV and COBI were derived from plasma concentration versus time data. The pharmacokinetic parameters to be assessed included:

| | |
|---|---|
| Cmax | Maximum observed plasma concentration |
| Tmax | Time of maximum observed plasma concentration |
| AUC(0-T) | Area under the plasma concentration-time curve from time zero time of last quantifiable concentration |
| AUC(INF) | Area under the plasma concentration-time curve from time zero extrapolated to infinite time |
| C24 | Observed concentration at 24 hour post-dose (for ATV only) |
| T-HALF | Apparent terminal half-life |

Individual subject pharmacokinetic parameter values were derived by non compartmental methods by a validated pharmacokinetic analysis program. Actual times were used for the analyses.

Table 6 lists the sampling schedule followed for the assessment of pharmacokinetics.

**Table 6: Pharmacokinetic Sampling Schedule**

| Study Day | Time (Event) Hour | Time (Relative To Dosing) Hour: Min | PK Blood Sample For ATV | PK Blood Sample For COBI |
|---|---|---|---|---|
| 1, 8, 15, 22 and 29 | 0 (predose) | 00:00 | X | X |
| | 1 | 01:00 | X | X |
| | 2 | 02:00 | X | X |
| | 2.5 | 02:30 | X | X |
| | 3 | 03:00 | X | X |
| | 4 | 04:00 | X | X |
| | 5 | 05:00 | X | X |
| | 6 | 06:00 | X | X |
| | 8 | 08:00 | X | X |
| | 12 | 12:00 | X | X |
| | 16 | 16:00 | X | X |

**Table 6: Pharmacokinetic Sampling Schedule**

| Study Day | Time (Event) Hour | Time (Relative To Dosing) Hour: Min | PK Blood Sample For ATV | PK Blood Sample For COBI |
|---|---|---|---|---|
| 2, 9, 16, 23, and 30 | 24 | 24:00 | X | X |
| | 30 | 30:00 | X | |
| | 36 | 36:00 | X | |
| 3, 10, 17, 24 and 31 | 48 | 48:00 | X | |

The plasma samples were analyzed for ATV and COBI by validated LC-MS/MS assays, the details of which are known to those skilled in the art.

To demonstrate bioequivalence for ATV between the test (Treatment B) and reference formulations (Treatment A), a linear mixed effects model were applied to the natural logarithms of ATV Cmax, AUC(0-T), and AUC(INF) with treatment, period and sequence as fixed effects and subject(sequence) as a random effect. Point estimates and 90% confidence intervals for differences on the log scale were exponentiated to obtain estimates for ratios of geometric means on the original scale. Bioequivalence was concluded if the 90% confidence intervals for the ratios of geometric means of the test formulation (FDC tablet of 300/150 mg ATV/COBI) to the reference formulation (300 mg ATV capsule co-administered with 150 mg COBI) were contained within 0.80 to 1.25 for ATV Cmax, AUC(0-T), and AUC(INF).

A similar linear mixed effect model was used to assess the relative bioavailability of ATV under fasted conditions (Treatment D versus Treatment C). Point estimates and 90% confidence intervals for ratios of geometric means were derived from the linear mixed effect model for Cmax, AUC(0-T), and AUC(INF).

In addition, a simplified linear mixed effect model with treatment as fixed effects and subject as a random effect was used to estimate the effect of a high fat meal and a light meal on the exposure of ATV when given as an FDC. Separate models were used for comparing the exposure when the FDC is given with a high fat meal versus fasted (Treatment E versus Treatment D), with a high fat meal versus with a light meal (Treatment E versus Treatment B), and with a light meal versus fasted (Treatment B versus Treatment D).

Similarly, a linear mixed effects model was applied to the natural logarithms of COBI Cmax, AUC(0-T), and AUC(INF) to compare Treatment B with Treatment A and to compare Treatment D with Treatment C. Point estimates and 90% confidence intervals for ratios of geometric means were provided for Cmax, AUC(0-T) and AUC(INF). The effect of a high fat meal and a light meal on the exposure of COBI were estimated in the similar way as described above for ATV exposure.

The results of the study demonstrated that all ATV PK parameter GMR 90% CIs fell within the predefined limits indicating bioequivalence of the FDC to ATV 300 mg and COBI 150 mg coadministered individually (Table 7 below). Although not prespecified, COBI in the FDC also met the criteria for bioequivalence to coadministration of the individual agents. Five subjects treated according to the protocol had total bilirubin elevations (1.2-2.1 × ULN), which were all improving or had resolved by study discharge.

A single dose of ATV 300 mg and COBI 150 mg was safe and well tolerated when administered either as the FDC or as individual agents.

**Table 7**

| Pharmacokinetic parameters | Adjusted geometric mean^{∗} | | |
|---|---|---|---|
| | ATV + COBI (Treatment A) | ATV/COBI FDC (Treatment B) | GMR (90% CI)^{∗} [B vs. A] |
| ATV, n | 63^{†} | 62^{†‡} | |
| Cₘₐₓ (ng/mL) (nanograms per milliliter) | 3822 | 4101 | 1.073 (1.012, 1.137) |
| AUC(INF) (ng.h/mL) (Hours times nanograms per milliliter) | 33475 | 35623 | 1.064 (1.011, 1.120) |
| AUC(0-T) (ng.h/mL) | 32723 | 34848 | 1.065 (1.012, 1.120) |
| COBI, n | 63^{†} | 62^{†‡} | |
| Cₘₐₓ (ng/mL) | 1320 | 1351 | 1.023 (0.991, 1.057) |
| AUC(INF) (ng.h/mL) | 9053 | 9225 | 1.019 (0.982, 1.058) |
| AUC(0-T) (ng.h/mL) | 8745 | 8912 | 1.019 (0.983, 1.057) |
| ^{∗}Based on a linear mixed effects model with natural logarithms of ATV or COBI as response and with treatment, period and sequence as fixed effects and subject (sequence) as a random effect. Point estimates and 90% confidence intervals (CI) for differences on the log scale were exponentiated to obtain estimates of geometric mean ratio (GMR) on the original scale. Bioequivalence was concluded if the B vs. A GMR 90% CIs fell within 0.80 to 1.25 for PK parameters. ^{†}One subject excluded as accidentally given both treatments A and B. ^{‡}One subject excluded as having vomited shortly after receiving treatment B. | | | |

Thus, disclosed herein is a tableted composition comprising atazanavir sulfate, cobicistat and a pharmaceutically acceptable carrier, said composition providing a blood concentration profile of atazanavir as measured by AUC(INF), that is from about 80% to 125% of 35623 ng.h/mL. Also disclosed herein is a tableted composition comprising atazanavir sulfate, cobicistat and a pharmaceutically acceptable carrier, said composition providing a blood concentration profile of atazanavir as measured by AUC(0-T) that is from about 80% to 125% of 34848 ng.h/mL. Also disclosed herein is a tableted composition comprising atazanavir sulfate, cobicistat and a pharmaceutically acceptable carrier, said composition providing a blood concentration profile of atazanavir as measured by Cmax that is from about 80% to 125% of 4101 ng/mL.

Disclosed herein is a tableted composition comprising atazanavir sulfate, cobicistat and a pharmaceutically acceptable carrier, said composition providing a blood concentration profile of cobicistat as measured by AUC(INF) that is from about 80% to 125% of 9225 ng.h/mL. Also disclosed herein is a tableted composition comprising atazanavir sulfate, cobicistat and a pharmaceutically acceptable carrier, said composition providing a blood concentration profile of cobicistat as measured by AUC(0-T) that is from about 80% to 125% of 8912 ng.h/mL. Also disclosed herein is a tableted composition comprising atazanavir sulfate, cobicistat and a pharmaceutically acceptable carrier, said composition providing a blood concentration profile of cobicistat as measured by Cmax that is from about 80% to 125% of 1351 ng/mL.

Disclosed herein are methods of treating HIV infection in a patient, which comprise administering to said patient a composition having the blood concentration profiles as described above.

## Claims

1. A bilayer tablet comprising atazanavir and cobicistat and a pharmaceutically acceptable carrier, said tablet comprising less than or equal to about 0.2% of degradant 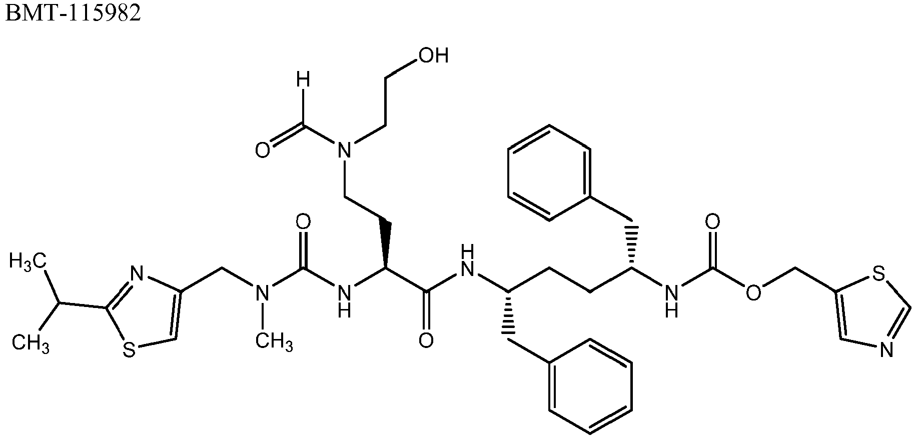 or

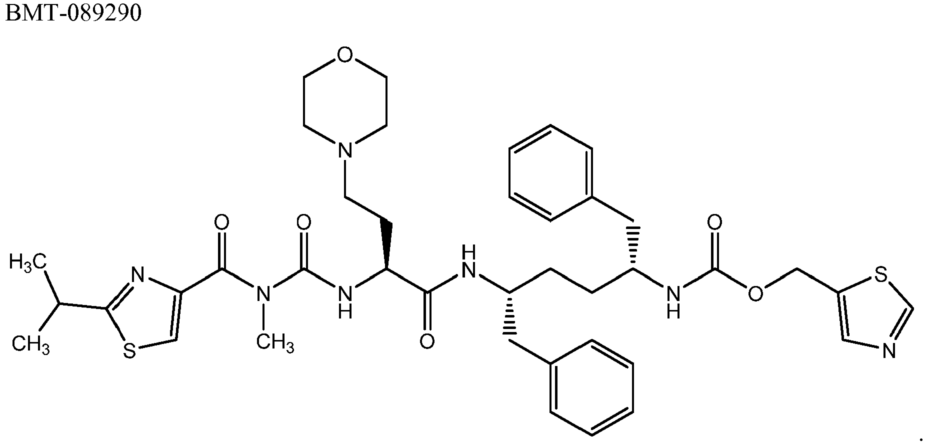

2. The bilayer tablet as defined in claim 1, wherein said tablet comprises less than or equal to about 4.0% total cobicistat impurities.

3. The bilayer tablet as defined in claim 2, wherein said tablet has less than about 2% total cobicistat impurities at about 12 months.

4. The bilayer tablet as defined in claim 3, wherein said tablet has less than about 1.4% cobicistat impurities at about 12 months.

5. The bilayer tablet as defined in claim 4, wherein said tablet does not crack upon release from a tablet press.

6. The bilayer tablet as defined in claim 1, wherein said tablet comprises less than about 3% total cobicistat impurities after 8 weeks at 40 ° C and 75% relative humidity.

## Patentansprüche

1. Zweischichttablette, umfassend Atazanavir und Cobicistat und einen pharmazeutisch verträglichen Träger, wobei die Tablette weniger als oder gleich etwa 0,2 % des Abbauprodukts BMT-115982 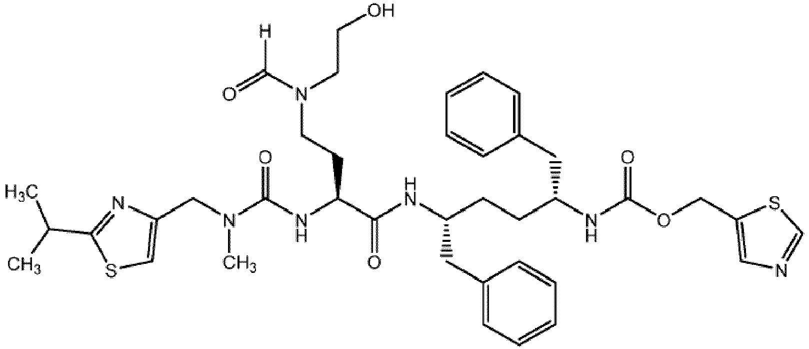 oder 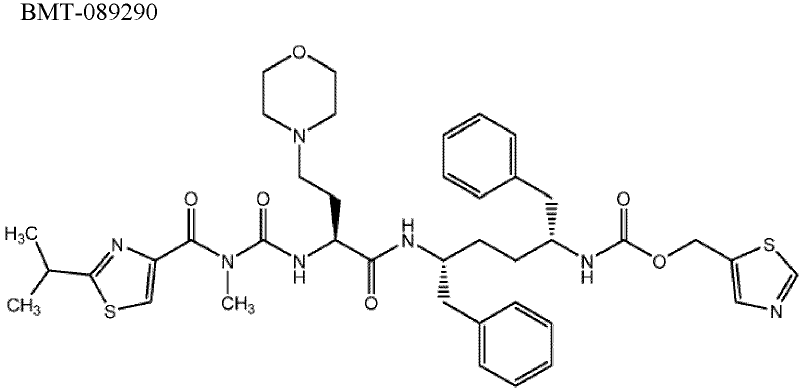 umfasst.

2. Zweischichttablette nach Anspruch 1, wobei die Tablette weniger als oder gleich etwa 4,0 % Gesamt-Cobicistat-Unreinheiten umfasst.

3. Zweischichttablette nach Anspruch 2, wobei die Tablette weniger als etwa 2% Gesamt-Cobicistat-Unreinheiten bei etwa 12 Monaten aufweist.

4. Zweischichttablette nach Anspruch 3, wobei die Tablette weniger als etwa 1,4 % Cobicistat-Unreinheiten bei etwa 12 Monaten aufweist.

5. Zweischichttablette nach Anspruch 4, wobei die Tablette bei seiner Ausgabe aus einer Tablettenpresse keine Risse bildet.

6. Zweischichttablette nach Anspruch 1, wobei die Tablette weniger als etwa 3 % Gesamt-Cobicistat-Unreinheiten nach etwa 8 Wochen bei 40 °C und 75 % relativer Feuchtigkeit umfasst.

## Revendications

1. Comprimé bicouche comprenant de l'atazanavir et du cobicistat et un support pharmaceutiquement acceptable, ledit comprimé comprenant un pourcentage inférieur ou égal à environ 0,2 % d'agent de dégradation BMT-115982 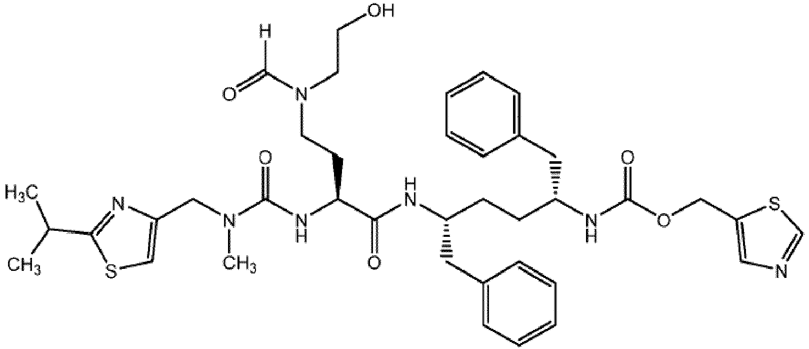 ou

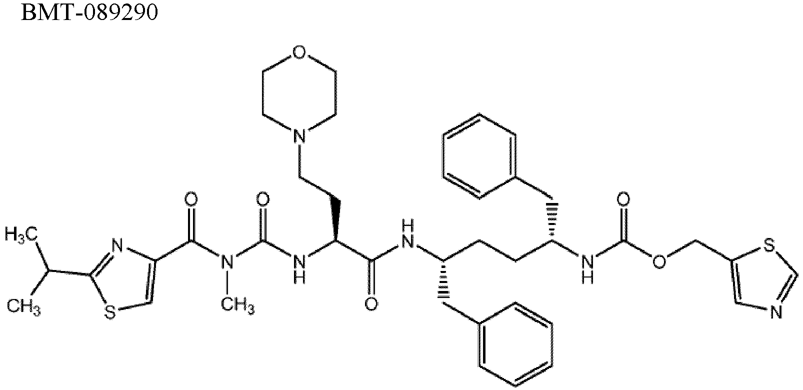

2. Comprimé bicouche selon la revendication 1, où ledit comprimé comprend un pourcentage inférieur ou égal à environ 4,0 % d'impuretés totales de cobicistat.

3. Comprimé bicouche selon la revendication 2, où ledit comprimé contient moins d'environ 2 % d'impuretés totales de cobicistat à environ 12 mois.

4. Comprimé bicouche selon la revendication 3, où ledit comprimé contient moins d'environ 1,4 % d'impuretés de cobicistat à environ 12 mois.

5. Comprimé bicouche selon la revendication 4, où ledit comprimé ne se fissure pas lors de sa libération d'une presse à comprimés.

6. Comprimé bicouche selon la revendication 1, où ledit comprimé comprend moins d'environ 3 % d'impuretés totales de cobicistat après 8 semaines à 40 °C et 75 % d'humidité relative.
